(19) Europäisches Patentamt
European Patent Office
Office européen des brevets

(11) EP 2 366 994 A1

(12) **EUROPEAN PATENT APPLICATION**

(43) Date of publication:
**21.09.2011 Bulletin 2011/38**

(51) Int Cl.:
***G01N 27/414*** *(2006.01)* ***G01N 33/543*** *(2006.01)*

(21) Application number: **10156924.2**

(22) Date of filing: **18.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **Knoll, Wolfgang**
**1220 Wien (AT)**

(72) Inventor: **Knoll, Wolfgang**
**1220 Wien (AT)**

(74) Representative: **Dey, Michael**
**Weickmann & Weickmann**
**Patentanwälte**
**Richard-Strauss-Strasse 80**
**81679 München (DE)**

(54) **Biosensor on thin-film transistors**

(57) The present invention relates to sensors, in particular, biosensors based on organic thin-film transistors (OTFT). The OTFT comprising a cover layer for coupling of molecules to be detected and for stabilising the transistor. The said layer covering at least part of the semiconductor layer of the OTFT.

EP 2 366 994 A1

## Description

The present invention relates to sensors, in particular, biosensors based on organic thin-film transistors.

Rapid and highly sensitive assays for biomolecules such as DNA or proteins have attracted enormous attention for a wide variety of applications ranging from genotyping to molecular diagnosis. For DNA analysis, however, e.g. conventional optical detection systems based on microarrays and real-time PCR involve expensive detection protocols, typically requiring a fluorescent dye and optical sources/detectors. Nevertheless, this method has become the standard technique for quantifying the extent of hybridization between surface immobilized probes and fluorophore-labeled DNA targets.

The development of a label-free, rapid, highly-selective and sensitive hybridization platform would have a significant impact on applications ranging from genomics sequencing to pathogen identification.

Recent advances in chemical detection research, in part benefitting from the overwhelming progress made in organic electronics, have shown great promise for a viable, low cost alternative to current optical detection systems (Roberts, M. E. et al. Water-stable organic transistors and their application in chemical and biological sensors. Proc. Natl. Acad. Sci. 105, 12134-12139 (2008)). The utilization of organic transistor technology in chemical sensors is particularly encouraging. This simple platform allows for the fabrication of low-cost, large-area, and flexible devices with air stability, low-power consumption, biocompatibility, and facile surface modification for the detection of a wide range of analyte species.

Although the performance of OTFTs (Organic Thin-Film Transistors) has improved remarkably during the last decade, the degradation of electrical performance in ambient air, water or buffer is a significant problem to realize commercial devices based on OTFTs. Therefore the trend of passivation on OTFT become common and widely investigated using single or multi-layers of organic/polymer materials.

However, the encapsulation of organic semiconductor-based electronics with thick layers leads to that changes on the surface due to the thick encapsulation coatings no longer result in a change in the transistor properties. Moreover, the problem is that the encapsulation layer would have to be functionalized in order to enable interactions with analyte molecules. The development of portable, robust OTFT based sensors functional in aqueous systems still faces many challenges.

Therefore, it was an object of the present invention to provide a sensor, in particular, a biosensor which is stable in measurement media such as water or puffer and, at the same time, has a biofunctionalized surface which is capable of interacting with molecules to be analyzed.

According to the invention, said object is achieved by a sensor comprising

(i) a transistor comprising

- (a) a gate layer,
- (b) a dielectric layer,
- (c) a semiconductor layer,
- (d) a source and
- (e) a drain, and

(ii) a layer providing for coupling of molecules to be detected and for stabilization of the transistor, which coupling/stabilization layer covers at least part of the semiconductor layer of the transistor.

The sensor according to the invention comprises a transistor which is at least partly covered with a coupling/stabilization layer. The coupling/stabilization layer allows for coupling or binding to molecules to be detected. Further, this layer stabilizes the transistor, in particular, when using the sensor in an aqueous or buffer medium. The invention therefore provides reliable and cheap biosensors which convert binding of a molecule to be detected, in particular, of a biomolecule such as DNA or proteins in real time into an electrical signal. The basis of said sensing mechanism is provided by a transistor, in particular, an organic thin-film transistor (OTFT). The coupling/stabilization layer provided by the invention allows the use of transistors, in particular, of OTFTs in aqueous or buffer media in which analytes such as biomolecules are usually provided. Destruction of the function of the electronics by an aqueous medium, as hitherto observed, is prevented by the coupling/stabilization layer according to the invention. In particular, it is prevented that the transistor loses rapidly and significantly its performance and that some of the layers are deteriorated or detach themselves, or their function is impaired.

On the other hand, the coupling/stabilization layer of the present invention allows binding and, thus, identifying of molecules to be detected, also in aqueous or buffer media. Finally, the function of the electronic component, i.e. the transistor, is not adversely affected by the inventive coupling/ stabilization layer.

Based on inexpensive electronic components, namely transistors, in particular, based on organic transistors

(OTFT), it is possible according to the invention, by applying a layer onto the semiconductor layer between source and drain electrode, to obtain both a molecular barrier as protective layer for the operation of the sensor in aqueous or physiological buffer solution and the functional layer required for specific recognition reactions between a capture or probe molecule and the analyte.

According to the invention, identification of bound analyte molecules and conversion of the binding into an electrical signal is enabled in the presence of the coupling/stabilization layer, above all, due to the fact that said layer preferably is very thin and has a thickness of preferably up to 800 nm, more preferably up to 500 nm, in particular, up to 300 nm, even more preferably up to 100 nm, in particular, up to 50 nm and most preferably up to 20 nm. The coupling/stabilization layer preferably has a thickness of at least 1 nm, more preferably, at least 2 nm, in particular, at least 5 nm and even more preferably at least 10 nm.

Application of such thin layers can be effected by spin-coating. Preferably, however, the layers are applied according to the invention by plasma deposition, in particular, by plasma-enhanced chemical vapor deposition. By applying the coupling/stabilizing layer via plasma polymerization, very thin layers can be applied and the function of the electronic component is impaired neither during the application process nor by the applied thin coating. By plasma polymerization, preferably by plasma polymerization in vacuum, layers can be applied onto transistors, in particular, on OTFTs, without the sensitive materials of the OTFTs being damaged thereby.

At the same time, by applying the coupling/stabilization layer by means of the inventive methods and, in particular, by plasma deposition, preferably by plasma-enhanced chemical vapor deposition, and even more preferably by plasma polymerization, it is possible to incorporate coupling groups into said layer concurrently with the production of the stabilization layer. Alternatively, these methods allow to crosslink not all reactive groups in the polymerization process, so that part of the reactive groups are retained, to which probe molecules or capture molecues then can be bound. Binding of the capture or probe molecules is preferably covalent.

In a preferred embodiment of the invention, for example, the coupling/ stabilization layer can be obtained by first forming an organic polymeric layer via plasma polymerization, with said layer subsequently being biofunctionalized. For biofunctionalization, conventional wet chemical methods can be employed in order to achieve specific sensitivity of the sensor, e.g. for specific DNA sequences, for specific antibodies or for the detection of specific marker substances.

The inventive sensors offer a number of advantages. For example, the coupling/stabilization layers can be designed in such a way that all common mechanism for specific detection can be employed such as sequence-specific hybridization between a transducer-immobilized capture DNA strand and the analyte DNA sequence complementary thereto from a test solution or binding of an antigen analyte, e.g. a specific cancer marker, to the corresponding antibody which is fixed in the coupling/stabilization layer.

A particular advantage of the sensor of the present invention is that analytes can be detected without the need for a label or marker molecule attached to the analyte. Thus, completely label-free detection can be performed.

The sensor according to the invention further allows for detection in situ and in real time. Thus, besides qualitative or quantitative determination of an analyte, also binding affinities between two binding partners and the corresponding kinetic reaction rates can be determined and quantified. Quantitative determination of analyte concentration in a test solution is possible, in particular, by using the electrical signals measured before, during and after binding. The resulting electronical signal changes can be recorded very fast. Further, the electrical measurement signal can be further processed easily, e.g. by a computer.

The nature of the inventive sensors furthermore allows parallel readout of many analyte determinations carried out simultaneously on accordingly functionalized measurement elements of a whole array of transistors in multiplex mode.

The employed components from organic electronics (plastic electronics) allow extremely cheap production, whereby the individual sensors can be provided as disposable measurement chips.

In one embodiment, the stabilization layer is applied by spin-coating. Preferably, perfluorocarbon is used as layer material for said method. An especially preferred coupling/stabilization layer applied by spin-coating comprises CYTOP (Cyclic Transparent Optical Polymer), a completely fluorinated polymer made from $CF_2 = CF\text{-}O\text{-}CF_2\text{-}CF_2\text{-}CF = CF_2$ having the formula:

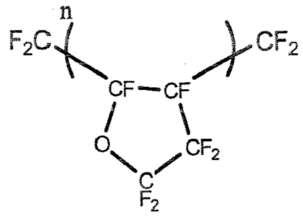

CYTOP

with n = the chain length of the polymer, in particular, n = 3-1000, in particular, 10-500.

For spin-coating, solutions of the material to be applied are prepared, e.g. solutions of a perfluorocarbon, e.g. solutions containing 1 to 10 wt.% of the material to be applied. Coating by means of spin-coating is then effected, for example, at 500 - 1500 rpm, in particular, at 700 - 1300 rpm for 10 seconds to 3 minutes, in particular, for 30 seconds to 1 minute, as a result of which layers in nanometer range, in particular, layers having a thickness of 1-800 nm, preferably of 100 - 500 nm, are obtained. Said layers can then be baked, e.g. at 70 - 90 °C for 20 minutes to 1 hour and, subsequently, at 110 °C to 150 °C for 1 - 5 hours.

The detecting component of the coupling/stabilization layer can be applied in the case of the spin-coating process by incorporating it into the solution to be applied, as a result of which it is applied also in the spin-coating process. Alternatively, it is possible to functionalize, e.g. to biofunctionalize the applied layer subsequently.

In a preferred and especially favorable embodiment of the invention, formation of the coupling/stabilization layer is effected by plasma deposition. Thereby, plasma deposition can be effected in a CW plasma, or preferably, in a pulsed plasma. Preferably, plasma deposition is plasma-enhanced chemical vapor deposition and, in particular, plasma polymerization. As starting materials for forming the coupling/stabilization layer by plasma deposition, in particular, monomers such as organic monomers as well as perfluoromonomers are used. Particularly suitable monomers, for example, are maleic acid anhydride, resulting in plasma polymerized maleic anhydride (ppMA), or allylamine, resulting in pp-allylamine films. Further suitable organic monomers are ethylene glycol precursors such as di(ethylene glycol) monovinyl ether, triethylene glycol monoallyl ether, tetraethylene glycol dimethyl ether, triglyme, tetraglyme or 2-hydroxyethyl methacrylate. Further suitable organic monomers for plasma assisted deposition are acrylic acid, acetic acid, propionic acid and their derivatives as well as aldehydes or anhydrides.

It is also possible to use siloxane such as hexamethyl disiloxane as monomers.

Particularly suitable perfluorocarbon monomers are compounds having from 1 to 30, in particular, from 1 to 10 carbon atoms. The compounds can be linear, branched or cyclic. Particularly suitable are e.g. PFDMCH monomer (perfluoro-1-3-dimethylcyclohexane), resulting in plasma polymerized PDFMCH films (ppPFDMCH), or tetrafluoromethane, octafluorocyclobutane, tetrafluoroethylene, perfluorooctane or perfluorocyclohexane.

In the case of plasma deposition it is also possible to form mixed films of organic monomers and perfluorinated monomers. Plasma polymerization is advantageous over wet chemistry deposition in that it is a single-step room temperature process. Further, no toxic or otherwise detrimental molecules, which may be necessary for wet application processes, are contained. Moreover, very thin coatings down to 1 nm still having good mechanical durability and good adhesive properties can be obtained. Continuos wave as well as pulsed plasma can be used. Plasma polymerization conditions suitable for passivation and surface functionalization of transistors include e.g. a process pressure of 0.01 to 0.2 mbar, a P of 1 to 300 W, in particular, 10 to 100 W, and an application time of from 5 seconds to 10 minutes. Pulsed plasma can have microsecond to millisecond pulses, in particular, pulses from 0.1 ms to 10 ms. Unless already done in the plasma polymerization step, the layer then can be functionalized by incorporating coupling molecules into the layer. Said incorporation, for example, can be effected by covalent binding to double bonds not yet reacted in the polymerization reaction. As coupling molecules, preferably molecules are incorporated which are specific for an analyte, for example, hybridization probes for DNA detection, antibodies for antigen detection, antigens for antibody detection or other specific coupling molecules.

Before applying the coupling/stabilization layer, the semiconductor layer of the transistor may be pretreated. Such preteatment may include cleaning, in particular, to prevent contamination which may ultimately lead to adhesion failure. Preferred methods of cleaning include plasma treatment using an oxygen/argon mixture or rinsing with a solvent and subsequent drying, e.g. drying in an oven or under dry nitrogen. In general, plasma polymerized films can be deposited directly onto the semiconductor layer. In some embodiments, however, an additional surface pretreatment is preferred, in particular, to improve adhesion and to prevent delamination, in particular, for applications requiring submersion of the device in aqueous solutions, in other solvents or in different pH or ionic environments. A surface pretreatment or precoating may be formed by self-assembled monolayers. Self-assembled monolayers can be used to initiate

chemical bonding to the semiconductor layer material on the one side and to the plasma polymerized film on the other side.

A further suitable surface pretreatment includes surface modification by an activating gas prior to plasma deposition. This pretreatment may act as cleaning process and at the same time for inducing surface roughening.

A further suitable pretreatment is the application of gradient films in which chemical and/or physical properties change with depth. Such gradient films can be synthesized in a series of consecutive plasma processing steps, for example, using decreasing duty cycles or decreasing energy input. For example, gradient films synthesized from the same precursor using initially high power plasma conditions followed by continuously decreasing energy may allow for improved substrate adhesion.

The plasma may be generated with a 13.56 Mhz rf generator. In a pulse mode, preferably, the peak power is between 20 and 200 W, on-times are between 20-400 μs and off-times are between 5-400 μs.

The deposition temperature during plasma polymerization is preferably below 100 °C.

An especially preferred field of application for the inventive sensors is the detection of biomolecules, in particular, biomolecules in an aqueous medium. Due to the coupling/stabilization layer provided by the invention the sensor is stable also in aqueous media, in particular, in a physiologically aqueous solution such as a buffer solution.

The inventive sensors are stable, in particular, for at least one day, more preferably for at least 10 days and even more preferably at least 30 days. Stable means that they are applicable for the intended use. In particular, the inventive sensors can be used in a buffer solution containing water and up to 1 mol/l buffer compound such as salts, preferably at least 0.1 mol/l buffer compounds, even more preferably at least 0.5 mol/l buffer compounds. The inventive sensors also exhibit stability over a high pH range, in particular, from pH 2 to pH 11, in particular, from pH 5 to pH 9.

The transistor employed in the sensor of the present invention preferably is a thin-film transistor, especially preferably an organic thin-film transistor. OTFTs being organic or polymeric electronic devices (also so-called "plastic electronics") are a very cost-efficient basis for the production of a sensor. Based on OTFTs, in particular, inexpensive disposable sensors, especially disposable biosensors can be provided.

The employed transistor comprises a gate layer (a). For producing the gate layer, conventional gate materials can be used, for example, metal such as gold, silver or nickel, or an Si-doped material.

The gate either can be applied on a substrate or carrier or itself constitute the carrier for the transistor.

The dielectric layer (b) is positioned on top of the gate layer. While basically any dielectric material can be used, the material of the dielectric layer (b) is preferably an organic or polymeric layer. Preferably, a crosslinkable polymer dielectric layer is employed which has high stability towards air and moisture and which can be crosslinked at low temperature. Preferably, the polymer matrix for the dielectric layer (b) is poly(4-vinylphenol) (PVP). The hydroxyl groups of PVP are well-suited for crosslinking with ambient-stable crosslinkers such as 4,4'-(hexafluoroisopropylidene)diphthalic anhydride (HDA) or suberoyl chloride (SC). Thus, the dielectric layer particularly preferably comprises PVP-HDA or PVP-SC. Thin isolated films of a crosslinked polymer dielectric can be formed e.g. by spin-coating. Preferably, the dielectric layer has a thickness of 10 to 100 nm, in particular, of 15 to 30 nm. However, it is also possible to form dielectric layers from other materials, for example, from perfluorocarbon polymers such as CYTOP.

A semiconductor layer (c) is applied onto the dielectric layer (b) on the side opposite the gate layer (a). Said layer preferably also consists of organic or polymeric materials. Materials suitable for the semiconductor layer, for example, are pentacene, 5,5'-bis-(7-dodecyl-9H-fluoren-2-yl)-2,2'-bithiophene (DDFTTF), copper (II) phthalocyanine (CuPc) and copper (II)-1,2,3,4,8,9,10,11,15,16,17,18,22,23,24,25-hexadecafluoro-29H,31 H-phthalocyanine (FCuPC). DDFTTF is especially preferred.

The semiconductor layer preferably has a layer thickness of 5 to 100 nm, in particular, 10 to 30 nm. Using organic or polymeric semiconductors allows markedly cheaper production of the components compared with semiconductor layers of CMOS on the basis of Si.

Then, a source (d) and a drain (e) electrode are applied onto the semiconductor layer. These can consist of any conductive material, e.g. gold. The semiconductor layer (c) is coated with the inventive coupling/stabilization layer in at least one section between the source and the drain. Preferably, the entire transistor surface is provided with the coupling/stabilization layer.

Optionally, the source and drain electrodes can be provided first with a further protective layer, e.g. with a layer of silicon monoxide. To that end, silicon monoxide can be thermally evaporated on the source and drain electrodes, thereby forming especially layers having 10 to 100 nm, preferably 30 to 70 nm.

The sensors of the invention can be employed, in particular, as sensors for various life functions, for the diagnosis of diseases, in environment technology, in food quality control or for monitoring safety-relevant areas. Besides application in devices in biosensor technology and medical diagnostics, thus, a variety of other technical applications are also possible. For example, the sensors can also be used for process monitoring in food technology, in environmental analysis or in food tracking. Basically, commercially available low-cost electronic components can be functionalized with the bioaffine detection and protection layers according to the invention. This enables totally different applications than the currently used very expensive optical biosensor technology.

For example, the labelling of packings, for example, food packings, can lead to entirely new safety standards

in food quality control.

Further, by using biotransistors, totally new possibilities are conceivable for permanent control of the quality of air, water, food, etc. in daily consumption by private households. To that end, for example, a central readout station can be provided, to which the biotransistors automatically send their measurement signals for further analysis and registration.

Simple parallelization of many sensors on one detector array can be used for comprehensive control of environmental risks, bacterial invasion in hospitals, domestic surroundings or safety-relevant domains, e.g. detection of explosives or drugs.

The invention is further illustrated by the attached Figures and the Examples given below.

Fig. 1 schematically shows the structure of a sensor of the invention. The sensor is formed from a transistor comprising a gate layer, a dielectric layer, a semiconductor layer, a source and a drain, and a coupling/stabilization layer covering the source, the drain and part of the semiconductor layer. In the region where the coupling/stabilization layer covers the semiconductor layer the coupling/stabilization layer is biofunctionalized with antigens, to which the analyte, i.e. antibodies, can bind.

Fig. 2 shows a schematic representation of OTFT sensor fabrication. (A) Fabrication of OTFT with a PVP-HDA (20 nm) dielectric layer, DDFTTF organic semiconductor (15 nm), and source-drain (S-D) electrodes with a W/L of 80. The interdigitated regions of the S-D electrodes were covered with thermally evaporated $SiO_x$ and the entire device was functionalized with 5 nm ppMA and a PNA probe.

Figure 3. Electrical characteristics in ambient and aqueous conditions of a representative OTFT with 15 nm DDFTTF on 20 nm PVP-HDA and W/L of 80. (A) Output characteristics ($I_{DS}$ VS. $V_{DS}$) with various $V_G$. (inset) Transfer characteristics ($I_{DS}$ VS. $V_G$) with a constant $V_{DS}$ of -1 V in ambient conditions. (B) Transfer characteristics ($I_{DS}$ VS. $V_G$) with a constant $V_{DS}$ of -0.5 V under ambient conditions (black), immediately after the injection of phosphate buffer solution (blue) and after 210 min in buffer solution (red). (C) Output characteristics ($I_{DS}$ VS. $V_{DS}$) in buffer solution.

Figure 4. Kinetic measurements for hybridization (association and dissociation) processes between the PNA-15mer probe and three different target DNA-15mers: T2-MMO; T1-MM1; T3-MM2. The solid lines are theoretical calculations based on a Langmuir model. (A) OTFT current response ($I_{DS}/I_{DS\text{-}baseline}$) with time upon exposure to DNA sequences of T2-MM0 (black), T1-MM1 (blue), T3-MM2 (orange) for an OTFT sensor operating at constant bias ($V_G$ = -1 V, $V_{DS}$ = -0.5 V). (B) Fluorescence intensity with time upon exposure to Cy5-labeled DNA sequences: T2-MM0 (black), T1-MM1 (blue), T3-MM2 (orange) using SPR-SPFS. (C) Angular reflectivity (dashed line) and fluorescence intensity scans (dots) measured in equilibrium for T2-MM0, T1-MM1, and T3-MM2.

Figure 5. Titration curves for PNA-DNA hybridization using the DDFTTF OTFT sensor at constant bias ($V_G$ = -1 V, $V_{DS}$ = -0.5 V) with a flow rate of 300 μL/min. Solid arrows indicate the additions of target DNA solutions and open arrows indicate the exchange to buffer solutions. The solid red lines show the Langmuir fits. (A) OTFT sensor titration with the T2-MM0 DNA-15mer and (B) with the T1-MM1 DNA-15mer. (C) The relationship between the surface coverage (saturation response) and target concentration ($C_o$) for T2-MM0 (from Fig. 4A) and T1-MM1 (from Fig. 4B). The solid S-shaped curves correspond to the fit by the Langmuir Isotherm with affinity constants ($K_A$) of 7 X $10^8$ $M^{-1}$ and 9 X $10^6$ $M^{-1}$ for T2-MM0 and T1-MM1, respectively.

Figure 6. Reproducibility and regeneration of the DDFTTF OTFT sensors. The OTFT responses were measured using a constant bias ($V_G$ = -1 V and $V_{DS}$ = -0.5 V) and flow rate of 300 μL/min. (A) $\Delta I_{DS}$ measured for multiple PNA-DNA hybridization cycles with T2-MM0 targets (100 nM). After each cycle the substrate was regenerated using 10 mM NaOH. (B) $\Delta I_{DS}$ measured for multiple PNA hybridization cycles for 200 nM T1-MM1 and (C) 200 nM T3-MM2. The substrate was rinsed with buffer solution between trials. The increase in $\Delta I_{DS}$ in first 3 cycles related with the shift in baseline current, $I_o$. (D) Raw data of the multiple hybridization cycles with the T2-MM0 target (100 nM). Up-head solid arrows indicate the T2-MM0 injections, down-head open arrows indicate the rinsing step with buffer solution, and up-head open arrows show the regeneration with 10 mM NaOH.

## Examples

### Example 1

In situ, label-free DNA detection using an organic transistor sensor

### 1.1 Fabrication procedure for DDFTTF based OTFTs with PVP-HDA polymer insulator

A solution of poly(4-vinylphenol) (PVP) (MW 20,000 g/mol) was prepared with a cross-linking agent 4,4'-(hexafluoroisopropylidene) diphthalic anhydride (HDA) in a molar ratio of 10: 1 in propylene glycol monomethyl ether acetate (PGMEA). The solution was filtered through a 0.2 μm syringe filter and spincoated with a thickness of 22 nm onto highly doped $n^{++}$ Si (100) substrates ($R \leq 0.008$ ohm-cm) at rates of 7,000 rpm for 1 min on a spin-coater (Headway Research). Before spin-coating, the substrates were treated with UV-ozone (Jelight Model 42) for 20 min and blown dry with filtered nitrogen (Mykrolis). The substrates were then cured at 100°C for 2 h. Organic semiconductor, 5,5'-bis-(7-dodecyl-9H-fluoren-2-yl)-2,2'-bithiophene (DDFTTF)[3], film was deposited by thermal evaporation (Angstrom Engineering) at a rate of 0.3 - 0.5 Å/s under a pressure of $6.5 \times 10^{-7}$ mbar. The substrate temperature was controlled by heating a copper block during deposition and was kept stable at 90°C, to a thickness of 15 nm. The top-contact devices were completed with gold electrodes thermally evaporated at 1 nm/s with a rotating substrate. Electrode dimensions were defined by a shadow mask with a channel width (W) and length (L) of 4 mm and 50 μm, respectively. Finally a 50 nm layer of silicon monoxide ($SiO_x$) was thermally evaporated on the source-drain electrodes to reduce the influence of charge screening effect during the OTFT sensor operation in buffer solution.

### 1.2 Formation of a coupling/stabilization layer by pulse-plasma polymerization

The surfaces of the OTFTs were modified with polymerized maleic anhydride (MA) using plasma-enhanced chemical vapor deposition (PE-CVD). The PE-CVD process did not cause any damage to the OTFT. The plasma polymerization was carried out using maleic anhydride (MA) as precursor to give an ultra-thin film (5 nm) of poly-maleic anhydride (ppMA).

### 1.3. Biofunctionalization of the surface

Amine terminated PNA-15mers (P2-15mers) were attached to the ppMA layer on the OTFT surface, which were used as the selective probe to target DNA sequences. P2-15mer PNA probes have been shown to be effective in discriminating between single base differences in applications using short sequences. The carboxylic acid groups on the polymer surface were activated by circulating a solution containing 0.2 M N-ethyl-N'-(3-dimethylaminopropyl)-carbodiimide hydrochloride (EDC; Fluka) and 0.05 M N-hydroxysuccinimide (NHS; Fluka) in the flow cell at a constant rate of 300 μL/min for 20 min, followed by exposure to 1 μM PNA solutions for 3 hr, and then rinsing with buffer solution. The presence of PNA on the DDFTTF surface did not affect the transistor characteristics. The specificity of these sensors toward the target DNA was evaluated using the fully complementary (T2-MM0), one-base mismatched (T1-MM1), and two-base mismatched (T3-MM2) DNA sequences. The sequence strands for PNA and DNA are displayed below.

**(B)**

**PNA Probe**

P2-15mer 5'-NH2-OOOOOO TGT ACA TCA CAA CTA-3'

**Target DNAs for OTFTs**

| | |
|---|---|
| T2-MM0 | 3'-ACA TGT AGT GTT GAT-5' |
| T1-MM1 | 3'-ACA TGC AGT GTT GAT-5' |
| T3-MM2 | 3'-ACA TGC ACT GTT GAT-5' |

**Target DNAs for SPFS**

| | |
|---|---|
| T2-MM0/Cy5 | 3'-ACA TGT AGT GTT GAT-Cy5-5' |
| T1-MM1/Cy5 | 3'-ACA TGC AGT GTT GAT-Cy5-5' |
| T3-MM2/Cy5 | 3'-ACA TGC ACT GTT GAT-Cy5-5' |

### 1.4 In situ detection of DNA hybridization

In an OTFT-based detection system, the occurrence of a chemical or physical absorption is converted to a current response ($\Delta I_{DS}$), which ultimately depends on the analyte composition, concentration and the OTFT bias conditions. For negatively-charged DNA targets, a decrease in current upon hybridization is observed due to the introduction of charges at the semiconductor surface. Stronger binding, or more efficient hybridization between the DNA and surface-bound PNA, should therefore result in a larger current change ($\Delta I_{DS}$). The electrical response of OTFTs to label-free DNA sequences was compared to the change in fluorescence intensity obtained with SPFS using flourophore-labeled DNA sequences on similar substrates.

In buffer solution, the baseline $I_{DS}$ was recorded after 100 s of constant bias ($V_{DS}$ = -0.5 V, $V_G$ = -1 V) prior to analyte injection. A flow rate of 300 μL/min was used to minimize mass transfer limitations of the analyte to the sensor surface. Our OTFTs experienced a slight drift in $I_{DS}$ with time, which can be attributed to gate bias stress. Between sensor trials on a given device, the initial current could be restored by removing the gate bias, or applying a reverse bias for a short time. Next, a 100 nM solution of T3-MM2 (two base mismatches) was injected and a decrease in $I_{DS}$ was observed. The $I_{DS}$ was measured as a function of time until the equilibrium between the bulk concentration ($C_o$) and the corresponding surface coverage ($\Theta(C_o)$) was achieved. The initial baseline current, $I_o$, was restored after a short rinse (~ 20 s) with buffer solution (Fig. 4A), indicating that T3-MM2 was either non-specifically bound or weakly hybridized with the PNA probe. A similar measurement with a solution of 100 nM T1-MM1 (one base mismatch) resulted in a larger decrease in $I_{DS}$, reaching equilibrium after 100 s. Rinsing with buffer solution resulted in only a slight recovery of $I_o$, indicating that some degree of hybridization, albeit weak, formed between T1-MM1 and the PNA probe (Fig. 4A). The OTFT drain current response to 100 nM solutions of T2-MM0 (DNA complement) showed the largest decrease over a short time as a result of the greater extent of hybridization compared to the mismatch sequences. Only a very slow decay was observed upon rinsing with buffer solution. This is consistent with our initial hypothesis that the efficient hybridization with the complementary sequence, T2-MM0, would result in the largest $\Delta I_{DS}$ and highest stability toward the buffer rinse (Fig. 4A). In each case, a rapid drop in the $I_{DS}$ was observed within the initial 10 s of buffer solution exchange owing to the removal of free DNA oligomers, which accounts for 0.75%, 11%, and 50% of the $I_{DS}$ for T2-MM0, T1-MM1, and T3-MM2, respectively.

SPFS measurements were performed to corroborate the OTFT response. The same three DNA targets were used with Cy5 fluorophores at their 5'-end, which could be excited by the evanescent field of the surface plasmon mode in SPFS. The SPFS experiments, carried out on Au substrates, also utilized PNA-15mer probes attached to a 5 nm ppMA film. Two typical optical measurements were performed: an angular scan of reflectivity and fluorescence intensity vs. incident angle (Fig. 4C) and a kinetic scan of fluorescence intensity vs. time during DNA hybridization (Fig. 4B).

The changes in fluorescence intensity measured as a function of time during the hybridization reaction can be used to determine the kinetic parameters, specifically the association ($K_{on}$) and dissociation ($K_{off.}$) rates. High photon counts in excess of $10^5$ cps were observed after rinsing the excess physisorbed DNA (Fig. 4B). The fluorescence angular scan (Fig. 4C) shows a pronounced enhancement in intensity at the resonant surface-plasmon excitation at a minimum reflectivity. Both figures show a clear discrimination in hybridization between the DNA complement and the two mismatched targets.

The kinetics of hybridization were determined from the change in $I_{DS}$ and fluorescence intensity. Based on the Langmuir model, the $\Delta I_{DS}$ as a function of time is described by a simple bimolecular reaction as follows

$$I_{Ci}(t) = (I_{max} - I_o)\left(1 - \exp\left(-\left(k_{on}C_o + k_{off}\right)t\right)\right) \qquad (1)$$

where $I_{max}$ *is* the equilibrium $I_{DS}$ following analyte exposure with a given concentration *($C_o$)*, and $I_o$ is the baseline current. Stable hybridization between T2-MM0 and the PNA probe resulted in very little dissociation through rinsing with buffer solution (60 s) and difficult quantification of $K_{off}$. By introducing a single or double base mismatch in the DNA sequence, the hybridized PNA-DNA complex becomes destabilized, enhancing the dissociation during the buffer rinse. The time-dependent dissociation is described by the following equation.

$$I_{ci}(t) = (I_{max} - I_o)\exp\left(-k_{off}t\right) \qquad (2)$$

The calculated result of $K_{on}$, $K_{off}$ and the affinity constant ($K_A$) are summarized in Table 1. $K_A$ decreases two orders of magnitude by inserting a single base mismatch (out of 15) in the target sequence relative to the complementary sequence, T2-MM0. The introduction of a second mismatch leads to a further decrease in $K_A$ by another order of magnitude. The $K_A$ value for T3-MM2 is relatively high and can be attributed to the excitation of labeled DNA in the bulk solution by the evanescent tail.

Table 1. Rate constants determined using the Langmuir model

| **DNA (100 nM)** | **$K_{off}$ ($S^{-1}$)** | **$K_{on}$ ($M^{-1}s^{-1}$)** | **$K_A$ ($M^{-1}$)** | **(±) E** |
|---|---|---|---|---|
| **Organic thin-film transistors sensors** | | | | |
| T3-MM2 | $5.0 \times 10^{-2}$ | $9.0 \times 10^3$ | $1.8 \times 10^5$ | $5.0 \times 10^5$ |

(continued)

| DNA (100 nM) | $K_{off}$ ($S^{-1}$) | $K_{on}$ ($M^{-1}s^{-1}$) | $K_A$ ($M^{-1}$) | (±) E |
|---|---|---|---|---|
| T1-MM1 | $3.0 \times 10^{-2}$ | $3.3 \times 10^{4}$ | $1.1 \times 10^{6}$ | $3.0 \times 10^{6}$ |
| T2-MM0 | $1.0 \times 10^{-3}$ | $4.0 \times 10^{5}$ | $4.0 \times 10^{8}$ | $2.6 \times 10^{8}$ |
| **Surface-plasmon fluorescence spectroscopy sensors** | | | | |
| T3-MM2/Cy5 | $2.0 \times 10^{-3}$ | $1.0 \times 10^{3}$ | $5.0 \times 10^{5}$ | |
| T1-MM1/Cy5 | $9.5 \times 10^{-4}$ | $9.0 \times 10^{3}$ | $9.4 \times 10^{6}$ | |
| T2-MM0/Cy5 | $2.0 \times 10^{-4}$ | $2.9 \times 10^{5}$ | $1.5 \times 10^{9}$ | |

Surface titration measurements were performed by passing solutions of target DNA with increasing concentrations over an immobilized probe and monitoring the association and dissociation. T2-MM0 DNA solutions with concentrations of 1, 5, 10, 20, and 50 nM (thin arrows in Fig. 5A) were sequentially injected into the flow channel resulting in increasingly larger changes in $I_{DS}$ due to higher equilibrium surface coverages. Between injections, non-bound DNA was rinsed with buffer solution (thick arrows in Fig. 5A), in addition to a 15 s pulse of 10 mM NaOH to completely regenerate the probes. Titration with the single base mismatch DNA target (T1-MM1) was performed in a similar manner, as shown Fig. 5B. The association and dissociation phases were fit with Eq. 1 and 2, respectively, which are shown by the red solid line in Figs. 5A and B. The average affinity constants were found to be $K_A$ = 7 z 2 X $10^{8}$ $M^{-1}$ for T2-MM0 and $K_A$ = 9 z X 10 $M^{-1}$ for T1-MM1.

The Langmuir Isotherm model that relates $\Theta(C_o)$ to $C_o$ is described by the following equation.

$$I_{DS\,max}(C_o) = \Theta(C_o) = \frac{K_A C_o}{1 - C_o K_A} \qquad (3)$$

$\Theta$ ($C_o$) is estimated by scaling the detected signal intensity $I_{DS\,max}$ ($C_o$) to that of a fully saturated surface and $C_o$. The Langmuir Isotherm curve for T2-MM0 and T1-MM1 was obtained by plotting the equilibrium current response ($I_{DS}$) versus concentration, which is shown in Fig. 5C. A nonlinear steady-state fit using Eq. 3, allows for the determination of $K_A$, yielding affinity constants for T2-MM0 and T1-MM1 which were in good agreement with those obtained from the association/dissociation fitting of the titration analysis.

The $K_A$S calculated from the OTFT sensors were 3-8 times lower than those determined via SPFS. We attribute this to the increasing surface charge density upon hybridization with negatively-charged DNA. As $\Theta(C_0)$ increases, this surface charge density generates an increasingly repulsive Coulombic barrier against further binding. This effect is also prevalent in the SPFS measurements, but to a lower extent since no external electric field is present. Operating in high ionic strength buffer solutions or diluting the probe density of the sensor matrix could limit the influence of Coulombic repulsion on the hybridization kinetics; however, both of these conditions would adversely affect the sensor sensitivity. The repulsive Coulombic barrier was further confirmed by the titration analysis, where it was not possible to see further hybridization without adding a rinsing step between each additional target concentration (Figs. 5A and B). The 1 nM sensitivity observed to the complementary DNA (T2-MM0) is consistent with previously reported results using SPFS.

The Langmuir adsorption Isotherm, i.e. $\Theta(C_o)$ vs. $C_o$, shows remarkable discrimination between T2-MM0 and T1-MM1 (Fig. 5C). The dotted line indicates the concentration at which the kinetic experiments were performed, $C_o$ = $10^{-7}$ M, which is well above the half-saturation ($1/K_A$) for the target complementary sequence, T2-MM0. This concentration is intentionally near the $1/K_A$ of T1-MM1, 9.5 X $10^{-8}$ M, to achieve efficient discrimination. Accordingly, the T1-MM1 sequence was easily dissociated from the surface-bound PNA; however, the T2-MM0 sequence ($1/K_A$= 2.5 $X$ $10^{-9}$ M,) could not be removed by rinsing with buffer solution within the timescale of our experiments. The T3-MM2 sequence has a rather high $1/K_A$ of 5.5 $X$ $10^{-6}$ M, which explains the relatively low $\Delta I_{DS}$ observed in Fig. 4A and the rapid dissociation upon rinsing.

## Reproducibility of OTFTs sensors

The reproducibility of the OTFT sensors was determined by measuring multiple hybridization cycles separated by buffer solution rinsing. 6 hybridization cycles were measured with T2-MM0 on a single device, and 9 hybridization cycles were examined for each mismatch DNA target, T1-MM1 and T3-MM2 on another substrate. Fig. 6(A-C) displays the $\Delta I_{DS}$ upon hybridization for each cycle. In all cases, the initial hybridization trials showed a small change $I_D$*s* upon exposure to the target DNA; however, the sensor response ($\Delta I_{DS}$) became constant as the baseline current stabilized (i.e., the gate bias stress was no longer significant). It was not necessary to regenerate the surface-bound PNA probes

after the hybridization of T1-MM1 (200 nM) and T3-MM2 (200 nM) due to the base mismatches and efficient dissociation in buffer solution. For the trials with the complementary target, T2-MM0 (100 nM), the PNA probes were regenerated with 10 mM NaOH solution for 15 s (raw data is shown in Fig. 6D), followed by a brief rinse with buffer solution to prepare the surface for the following trial.

**Example 2**

**In-situ selective IgG detection using an aqueous stable pentacene-based transistor**

**2.1 Manufacture of OTFTs**

OTFTs were fabricated on a heavily doped silicon wafer followed by the bottom-gate and bottom-contact structures. The substrates were cleaned using acetone, iso-propanole, ethanol, and rinse with de-ionized water and then centered at 100oC for 5 min. Spin-coated CYTOP (15 nm) on thermally grown $SiO_2$ (200 nm) was used for gate dielectric insulator to decrease hysteresis and threshold voltage shift by removing trap site on surface. 1 wt % CYTOP solution was used to spin coat with 4000 rpm for 40 s in Ar-filled glove box. CYTOP film was baked at 800C for 30 min and 1800C for 1 hr. The surface roughness (rms) of the insulator was investigated by atomic force microscope (AFM). (NanoScope Dimension 3100 CL) i.e., 0.8 nm. Source-drain electrodes with a W/L of 10 were evaporated by using shadow mask. Furthermore Au electrodes were treated for 1 hr with 0.1 mM solution of 2-mercapto-5-nitrobenzimidazole (MNB) diluted in ethanol to enhance the charge carriers. Finally a 30 nm thick pentacene film was thermally evaporation under a pressure of 6.5 X 10-7 mbar with a deposition rate of 0.5 Å/s at 300C to control the growth of pentacene crystal.

Pulse plasma polymerization of perfluor-1-3-dimethylcyclohexam (PFDMCH) was carried out to passivate and maleic anhydride (MA) to functionalize the surface of each device (optical/electrical) for the selective hybridization process. In a PE-CVD reactor, a 13.56 MHz r.f. generator was capacitively coupled to the gas via an LC matching circuit and a copper coil (0.5 mm diameter, 10 turns) wound externally around the pyrex glass cylindrical reaction chamber, spanning 8-16 cm from the gas inlet. The substrate position inside the plasma chamber was optimized to achieve a homogeneous film with the required thickness. A signal generator was attached to the r.f. power source, and a cathode ray oscilloscope was used to monitor the pulse duration, interval and amplitude. Thiol containing 2-Mercapto-5-Nitrobenzimidazole (MNB) (molecular structure is given in Fig. SI2) was used as an adhesive mono-layer, diluted in ethanol with 5 mM concentration (immersion time ~30 min), prior to the plasma polymerization of MA on optical device.

Characterizations of the chemical structure of thin-films deposited by PE-CVD were carried out using Fourier Transform Infrared Spectroscopy (FT-IR) (Nicolet 850 spectrometer) in the reflection mode and X-ray Photoelectron Spectroscopy (XPS) (Physical Electronics 5600 A instrument) with a Mg KR (1253.6 eV) X-ray source operating at 300 W. A pass energy of 117.40 eV was used for the survey spectra. The spectra were recorded using a 45° take-off angle relative to the surface normal and the scans were analyzed using the MultiPak 5.0 software.

For biosensing measurements, all bio-chemical molecules were diluted in freshly prepared sodium acetate buffer solution (ABS) (10 mM concentrated with pH 7). Antigen [Bovine Serum Albumin (BSA)] (MW ~66kDa) was purchased from Sigma Aldrich and antibody (antiBSA) IgG from Millipore. We used a flow cell made of Plexiglas with an inlet and an outlet opening (diameter = 1 mm) on top, confining the total volume to ~30 $mm_3$. The contact areas between substrate and flow cell were sealed by Viton O-ring. The flow cell was connected to a peristaltic pump (Novo-Chem) and the sample reservoir using Tygon tubes with an inner diameter of 0.76 mm. The peristaltic pump maintained a constant flow speed of 300 μL/min to ensure a constant analyte concentration.

The flow cell was adhered to the substrates such that the solution chamber mainly contacted the channel region of the OTFTs. We observed an increase in on current when the device was measured in a freshly prepared sodium acetate buffer solution compared to ambient air at low operating voltages -5 V, which is necessary for stable operation in water. The slight variation in the electrical characteristics illustrates relatively stable behavior with a small change in mobility from 0.006 cm2/Vs in ambient air to 0.0052 cm2/Vs in buffer solution.

**2.2 In-situ anti-BSA (IgG) hybridization reactions by OTFT**

Antigen/antibody interactions belong to a large group of non-covalent biological binding reactions which depend mainly on structural complementarity between a ligand and a binding site (receptor) on a macromolecule.

To investigate the effect of net charges on the transistor sensor surface due to the binding BSA/IgG, a 500 nM concentrated IgG diluted in buffer solution at pH 7 was injected and the decrease in $I_{DS}$ was recorded as a function of time until the equilibrium between the bulk concentration and the corresponding surface coverage was reached, we switch to buffer solution, which however displays a decay (increasing $I_{DS}$). This rinsing process was continued for 130 s to observe IgG dissociation behavior, we further regenerate the surface with a 15 s pulse of Glycine (10 mM, pH 2) solution and continued to exposing the sensor surface with same concentration of IgG i.e., 500 nM diluted in a buffer

solution at pH 5. The $I_{DS}$ start to increase and sequentially after exchanging IgG with buffer solution we note a decrease in $I_{DS}$. Thus, we observe two kind of effects on $I_{DS}$ signal, a) at pH 7 the (change in current) $\Delta I_{DS}$ was 5 time smaller than at pH 5, b) the sign of polarity was opposite at pH 7 compare to pH 5. These experimental results are consistent with our initial hypotheses that above isoelectric point (pI), IgG behaves as a negatively charges and below this point the positive behaviors would be dominant. Further, these results also indicate that transistor shows an ideal p-type characteristic.

## Claims

**1.** A sensor comprising

(i) a transistor comprising

(a) a gate layer,
(b) a dielectric layer,
(c) a semiconductor layer,
(d) a source and
(e) a drain, and

(ii) a layer providing for coupling of molecules to be detected and for stabilization of the transistor, which coupling/stabilization layer covers at least part of the semiconductor layer of the transistor.

**2.** The sensor according to claim 1, wherein the coupling/stabilization layer has a thickness of from 1 nm to 800 nm, in particular, from 5 nm to 50 nm.

**3.** The sensor according to claim 1 or 2, wherein the coupling/stabilization layer is applied by plasma deposition, in particular, by plasma-enhanced chemical vapor deposition and/or by spin-coating.

**4.** The sensor according to any of the preceding claims, wherein the coupling/stabilization layer comprises polymaleic anhydride, polyPFDMCH and/or CYTOP.

**5.** The sensor according to any of the preceding claims, wherein the coupling/stabilization layer comprises molecular probes for a molecule to be detected, in particular, molecular probes for DNA or proteins.

**6.** The sensor according to any of the preceding claims for sensing biomolecules in an aqueous medium, in particular, in a buffer.

**7.** The sensor according to any of the preceding claims, wherein the transistor is an organic thin-film transistor.

**8.** The sensor of any of the preceding claims, being operable at a low voltage of ≤ 2 V.

**9.** The sensor of any of the preceding claims, wherein the dielectric layer is an organic layer, in particular, a layer of crosslinked PVP or/and CYTOP.

**10.** The sensor according to any of the preceding claims, wherein the semiconductor layer is an organic layer, in particular, a layer comprising DDFTTF, dihexyl FTTF, and/or pentacene.

Fig. 3.1

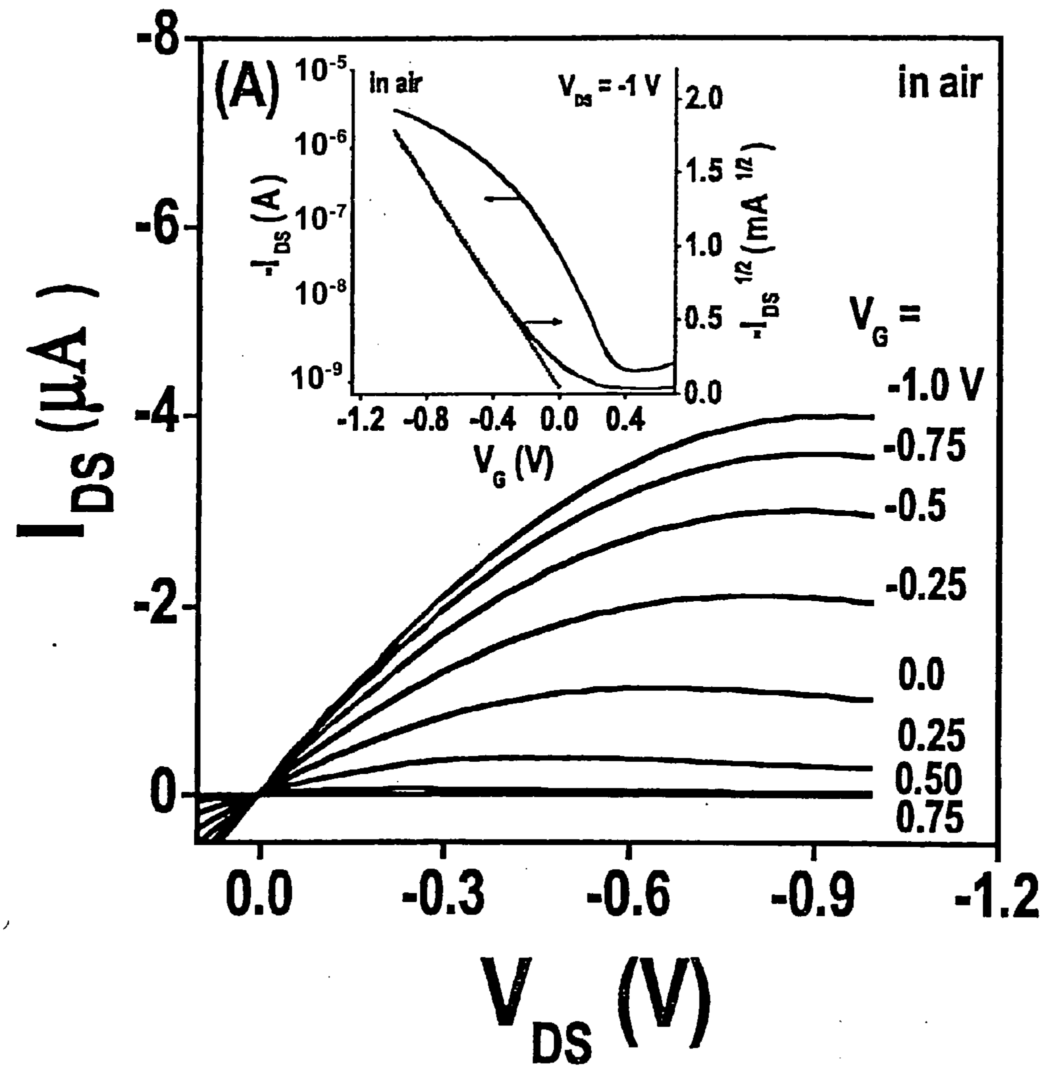
(A)
in air
in air
$V_{DS}$ = -1 V
$-I_{DS}$ (A)
$-I_{DS}^{1/2}$ (mA $^{1/2}$)
$V_G$ (V)
$V_G$ =
-1.0 V
-0.75
-0.5
-0.25
0.0
0.25
0.50
0.75
$I_{DS}$ (μA)
$V_{DS}$ (V)

Fig. 3.2

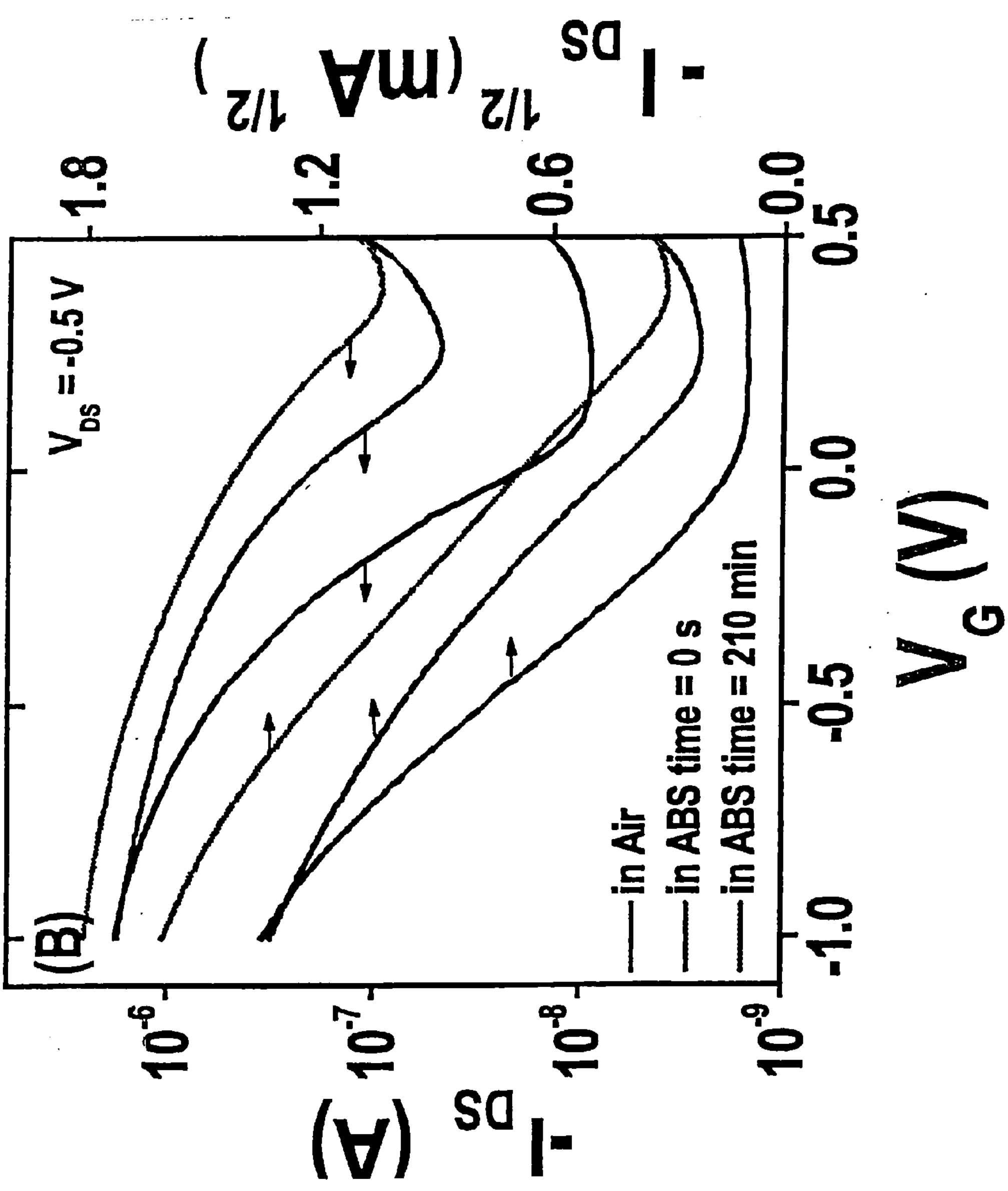
(B)
$V_{DS}$ = -0.5 V
in Air
in ABS time = 0 s
in ABS time = 210 min
$V_G$ (V)
-1.0
-0.5
0.0
0.5
$-I_{DS}$ (A)
$10^{-6}$
$10^{-7}$
$10^{-8}$
$10^{-9}$
$-I_{DS}^{1/2}$ ($mA^{1/2}$)
1.8
1.2
0.6
0.0

Fig. 3.3

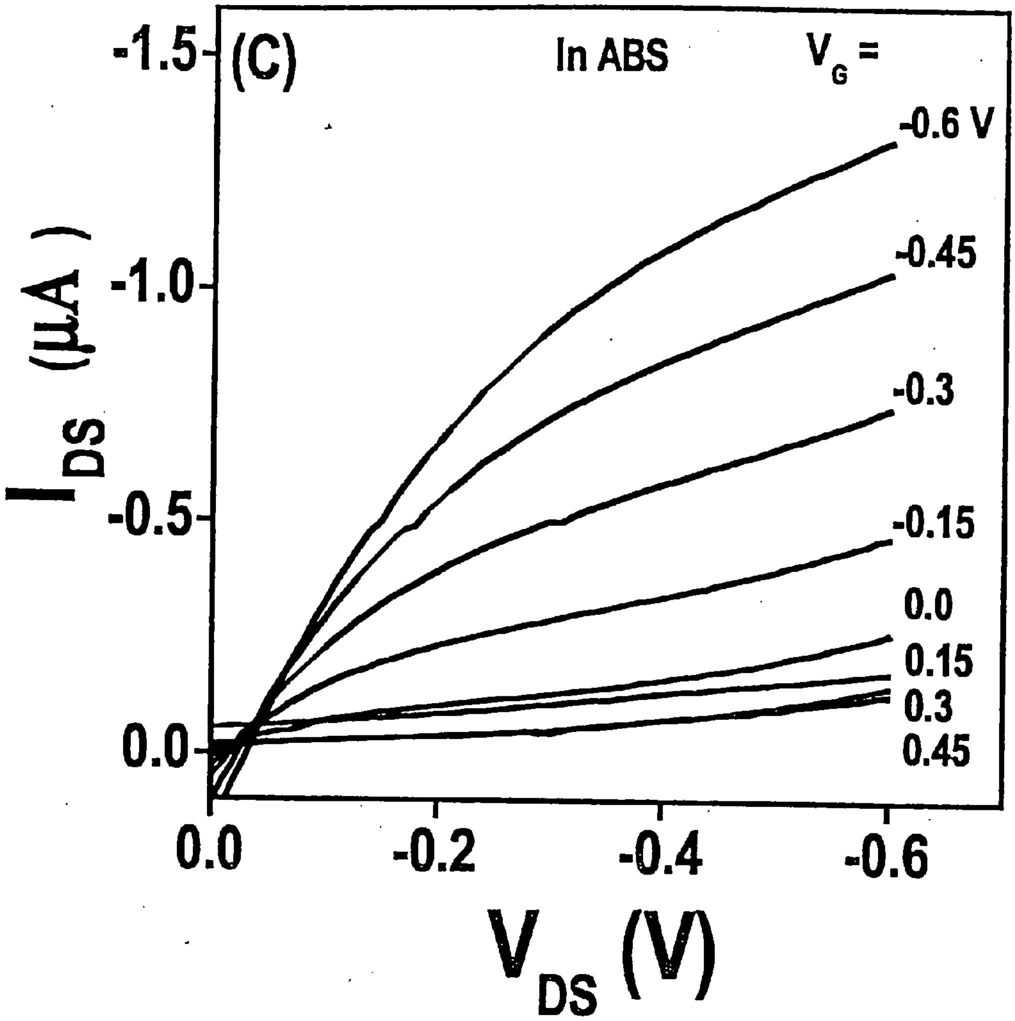
(C)
In ABS
$V_G$ =
-0.6 V
-0.45
-0.3
-0.15
0.0
0.15
0.3
0.45
-1.5
-1.0
-0.5
0.0
$I_{DS}$ (µA)
0.0
-0.2
-0.4
-0.6
$V_{DS}$ (V)

Fig. 4.1

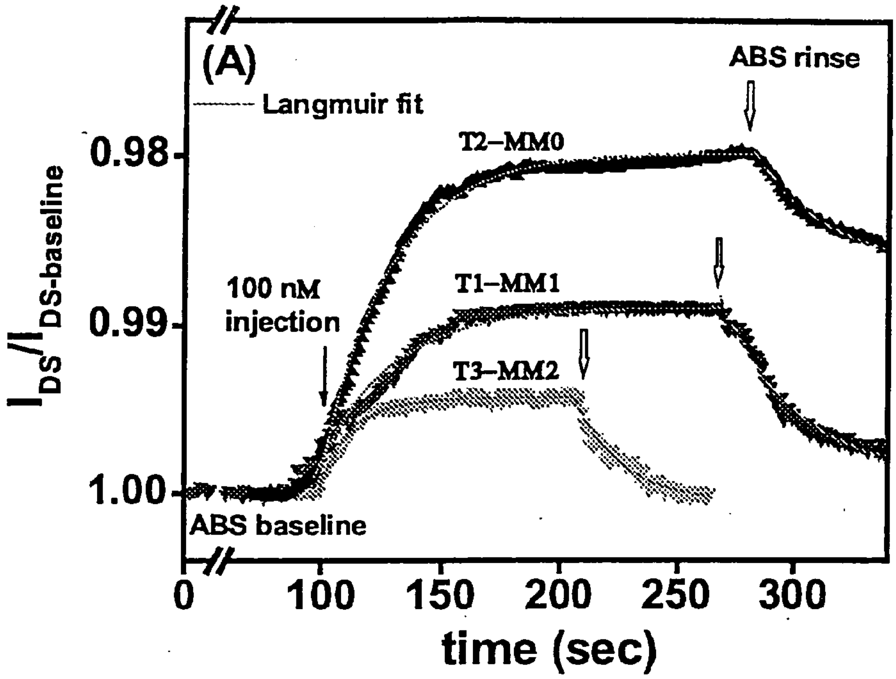
(A)
Langmuir fit
ABS rinse
T2–MM0
T1–MM1
T3–MM2
100 nM
injection
ABS baseline
$I_{DS}/I_{DS\text{-baseline}}$
0.98
0.99
1.00
0
100
150
200
250
300
time (sec)

Fig. 4.2

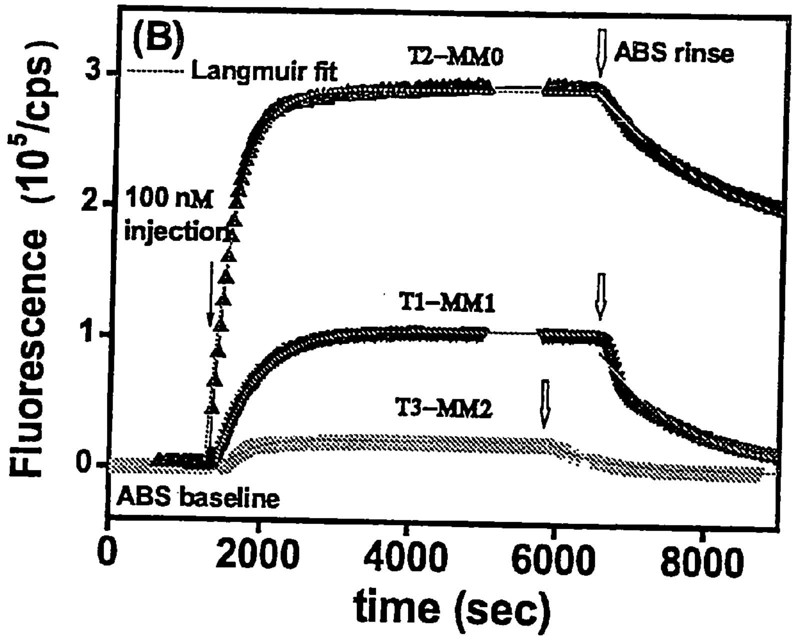
(B)
Langmuir fit
T2-MM0
ABS rinse
100 nM
injection
T1-MM1
T3-MM2
ABS baseline
Fluorescence ($10^5$/cps)
time (sec)
0
1
2
3
0
2000
4000
6000
8000

Fig. 4.3

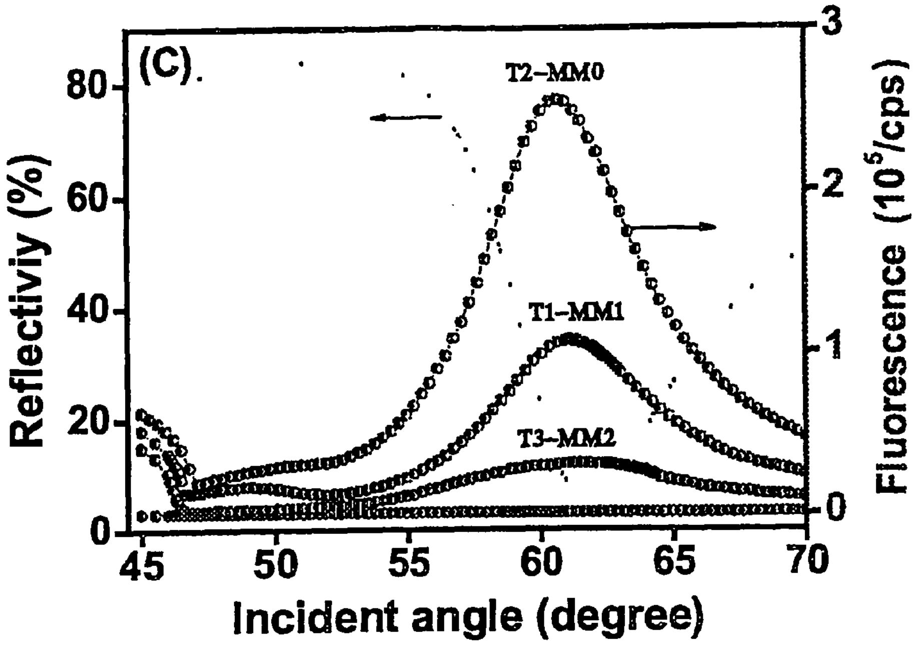
(C).
T2-MM0
T1-MM1
T3-MM2
Reflectiviy (%)
80
60
40
20
0
Fluorescence (10^5/cps)
3
2
1
0
45
50
55
60
65
70
Incident angle (degree)

Fig. 5.1

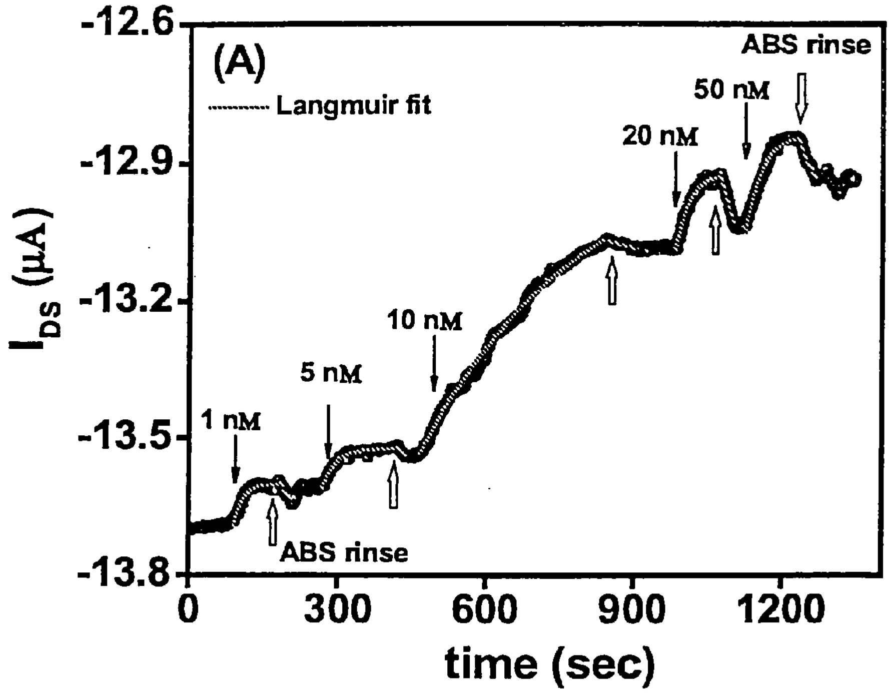
-12.6
-12.9
-13.2
-13.5
-13.8
$I_{DS}$ (μA)
0
300
600
900
1200
time (sec)
(A)
Langmuir fit
1 nM
ABS rinse
5 nM
10 nM
20 nM
50 nM
ABS rinse

Fig. 5.2

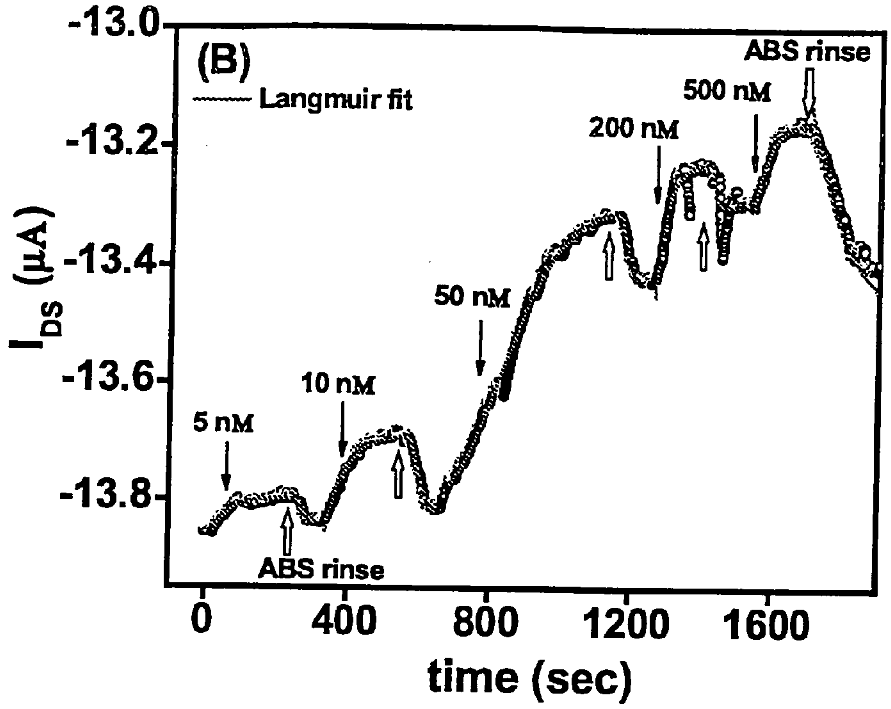
(B)
Langmuir fit
5 nM
ABS rinse
10 nM
50 nM
200 nM
500 nM
ABS rinse
-13.0
-13.2
-13.4
-13.6
-13.8
$I_{DS}$ (μA)
0
400
800
1200
1600
time (sec)

Fig. 5.3

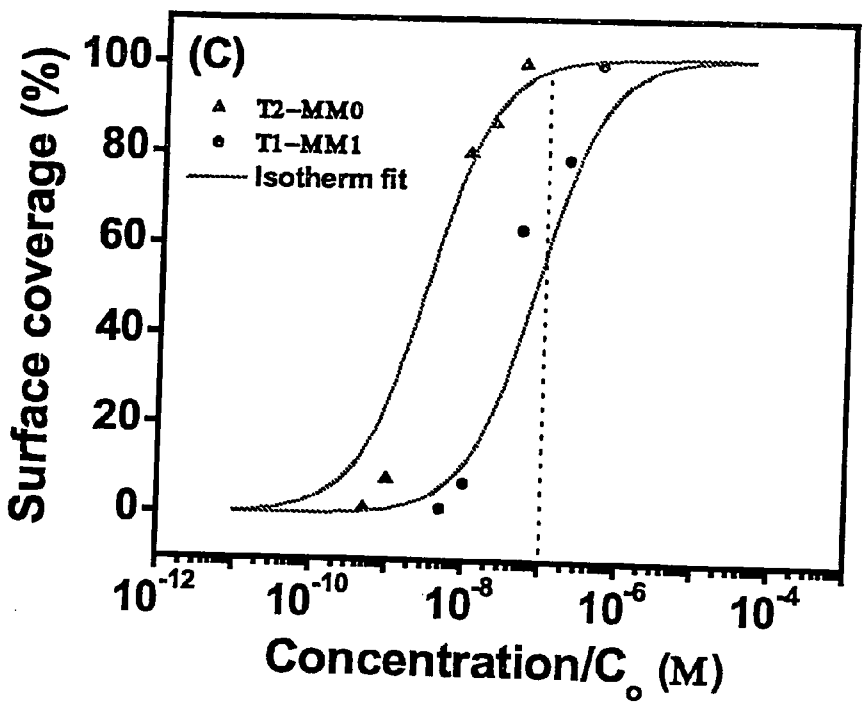
(C)
T2-MM0
T1-MM1
Isotherm fit
Surface coverage (%)
100
80
60
40
20
0
10-12
10-10
10-8
10-6
10-4
Concentration/Co (M)

Fig. 6.1

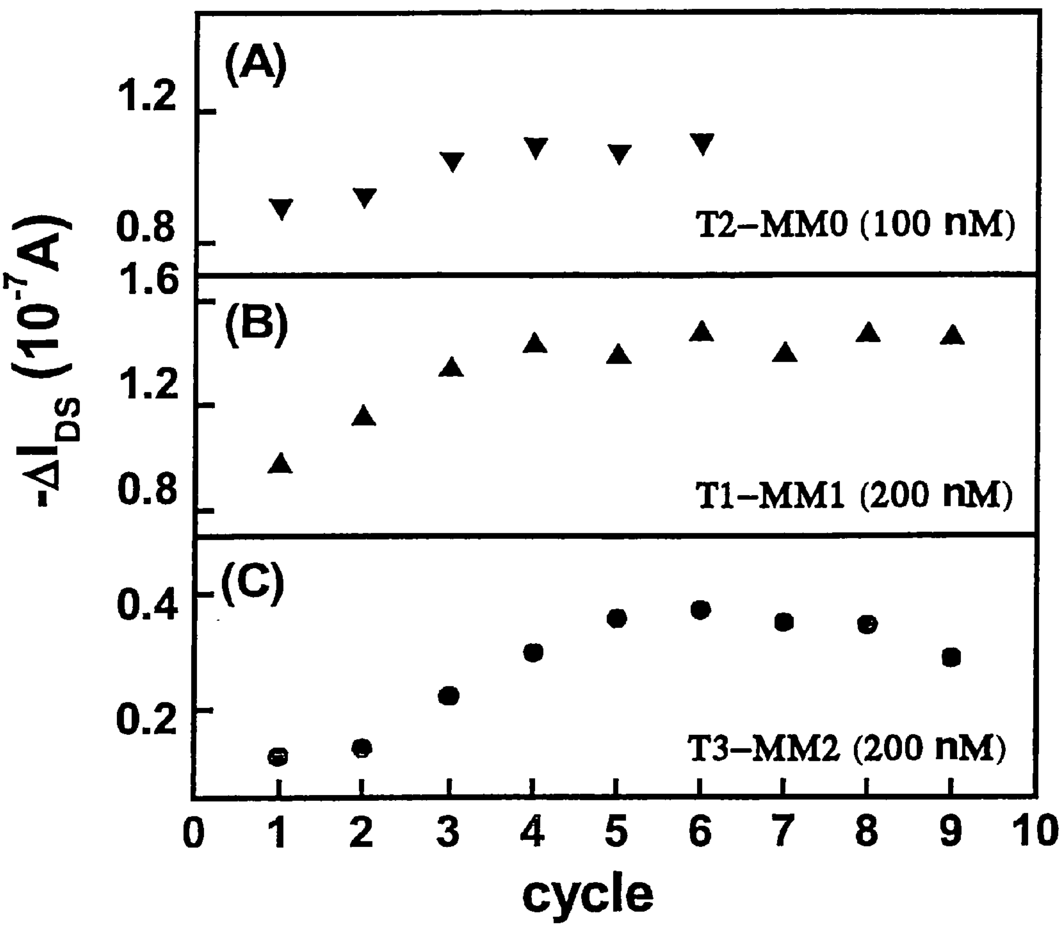
(A)
1.2
0.8
T2–MM0 (100 nM)
1.6
(B)
1.2
0.8
T1–MM1 (200 nM)
(C)
0.4
0.2
T3–MM2 (200 nM)
$-\Delta I_{DS}$ ($10^{-7}$A)
0
1
2
3
4
5
6
7
8
9
10
cycle

Fig. 6.2

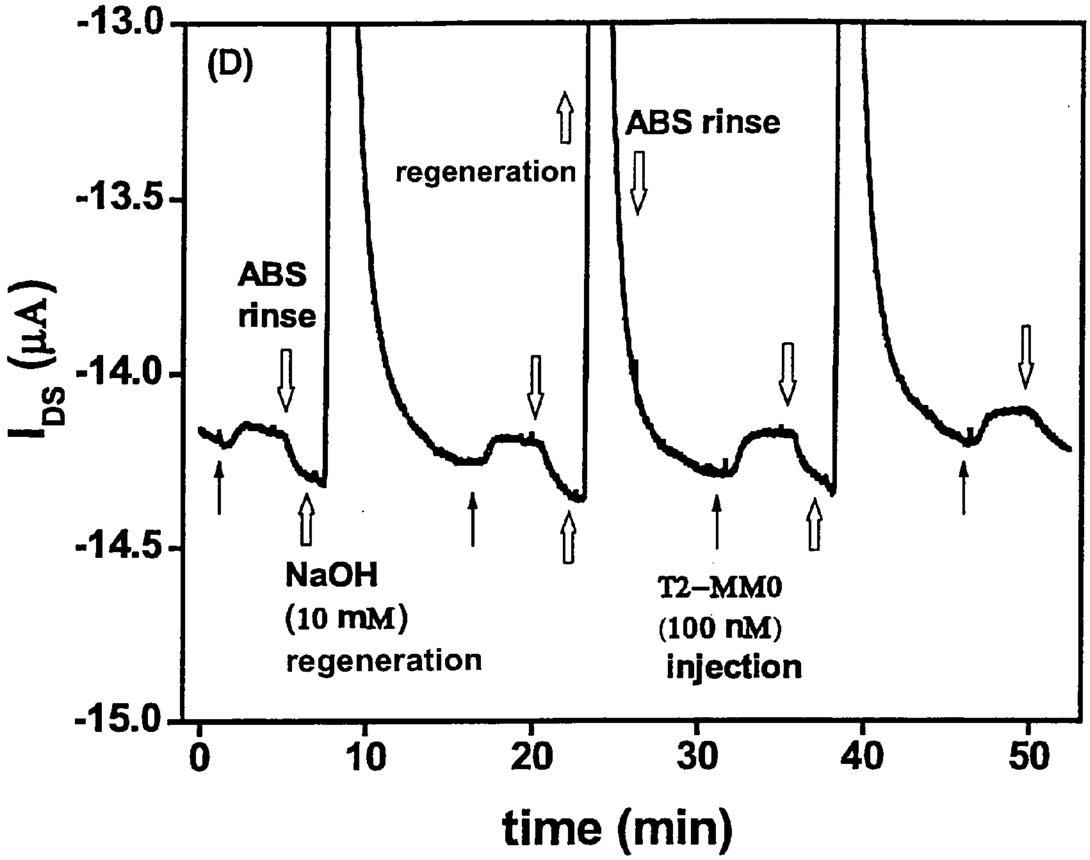

(D)
-13.0
-13.5
-14.0
-14.5
-15.0
$I_{DS}$ (μA)
0
10
20
30
40
50
time (min)
ABS rinse
regeneration
ABS rinse
NaOH
(10 mM)
regeneration
T2–MM0
(100 nM)
injection

Europäisches Patentamt
European Patent Office
Office européen des brevets

# EUROPEAN SEARCH REPORT

Application Number
EP 10 15 6924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/138506 A1 (KONINKL PHILIPS ELECTRONICS NV [NL]; SETAYESH SEPAS [NL]; DE LEEUW DAG) 6 December 2007 (2007-12-06)<br>* page 6, line 1 - line 6 *<br>* page 7, line 13 - line 22; figures 1,2 *<br>----- | 1-3,5-10 | INV.<br>G01N27/414<br>G01N33/543 |
| X<br><br>Y | EP 1 464 953 A1 (LUCENT TECHNOLOGIES INC [US]) 6 October 2004 (2004-10-06)<br>* paragraph [0014] - paragraph [0027]; figure 1 *<br>----- | 1-3,5-7,<br>10<br>8,9 | |
| Y | BARTIC C ET AL: "Field-effect detection of chemical species with hybrid organic/inorganic transistors"<br>APPLIED PHYSICS LETTERS, AIP, AMERICAN INSTITUTE OF PHYSICS, MELVILLE, NY, US<br>LNKD- DOI:10.1063/1.1527698,<br>vol. 82, no. 3,<br>20 January 2003 (2003-01-20), pages 475-477, XP012034609<br>ISSN: 0003-6951<br>* the whole document *<br>----- | 1-10 | TECHNICAL FIELDS SEARCHED (IPC)<br>G01N |
| Y | ROBERTS M E ET AL: "Material and device considerations for organic thin-film transistor sensors"<br>JOURNAL OF MATERIALS CHEMISTRY ROYAL SOCIETY OF CHEMISTRY UK,<br>vol. 19, no. 21,<br>11 February 2009 (2009-02-11), pages 3351-3363, XP002598728<br>ISSN: 0959-9428 DOI: DOI:10.1039/B816386C<br>* page 3354 - page 3357 *<br>-----<br>-/-- | 1-10 | |

The present search report has been drawn up for all claims

1

| Place of search | Date of completion of the search | Examiner |
|---|---|---|
| Munich | 31 August 2010 | Komenda, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 15 6924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG Q ET AL: "Label-free low-cost disposable DNA hybridization detection systems using organic TFTs" BIOSENSORS AND BIOELECTRONICS, ELSEVIER BV, NL, vol. 25, no. 5, 15 January 2010 (2010-01-15), pages 972-977, XP026808883 ISSN: 0956-5663 [retrieved on 2009-09-09] * abstract * ----- | 1-10 | |
| A | US 2009/278117 A1 (LIM SANG CHUL [KR] ET AL) 12 November 2009 (2009-11-12) * paragraphs [0031], [0041], [0045], [0046]; figure 6 * ----- | 1-10 | |
| | | | TECHNICAL FIELDS SEARCHED (IPC) |

The present search report has been drawn up for all claims

1

| Place of search | Date of completion of the search | Examiner |
|---|---|---|
| Munich | 31 August 2010 | Komenda, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 15 6924

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2010

| Patent document cited in search report | Publication date | Patent family member(s) | Publication date |
|---|---|---|---|
| WO 2007138506 A1 | 06-12-2007 | CN 101454659 A | 10-06-2009 |
| | | EP 2030007 A1 | 04-03-2009 |
| | | JP 2009539241 T | 12-11-2009 |
| | | US 2009267057 A1 | 29-10-2009 |
| EP 1464953 A1 | 06-10-2004 | CN 1540328 A | 27-10-2004 |
| | | CN 101487815 A | 22-07-2009 |
| | | JP 2004309483 A | 04-11-2004 |
| | | KR 20040086196 A | 08-10-2004 |
| | | US 2004195563 A1 | 07-10-2004 |
| | | US 2006243969 A1 | 02-11-2006 |
| US 2009278117 A1 | 12-11-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ROBERTS, M. E. et al.** Water-stable organic transistors and their application in chemical and biological sensors. *Proc. Natl. Acad. Sci.,* 2008, vol. 105, 12134-12139